# EUROPEAN PATENT SPECIFICATION

(11) **EP 2 346 423 B1**
(45) Date of publication and mention of the grant of the patent: **19.12.2012**
(21) Application number: 09791525.0
(22) Date of filing: 14.08.2009
(51) Int. Cl.: A61B 17/70

(54) **POSTERIOR DYNAMIC STABILIZATION SYSTEM**
POSTERIORES DYNAMISCHES STABILISATIONSSYSTEM
SYSTÈME DE STABILISATION DYNAMIQUE POSTÉRIEURE

(30) Priority: 14.08.2008 US 88910 P
(43) Date of publication of application: 27.07.2011
(73) Proprietor: Synthes GmbH, 4436 Oberdorf (CH)
(72) Inventor: OVERES, Thomas, 4513 Langendorf (CH); FRIGG, Robert, 2544 Bettlach (CH); LECHMANN, Beat, 2540 Grenchen (CH)
(74) Representative: Höhfeld, Jochen
(86) International application number: PCT/US2009/053841
(87) International publication number: WO 2010/019857

(56) References cited:
- EP-A- 0 677 277
- WO-A-01/56489
- WO-A-2006/066053
- WO-A-2007/103404
- US-A1- 2007 016 200
- US-A1- 2007 270 860
- US-A1- 2008 097 434

## Description

### BACKGROUND OF THE INVENTION

Spinal fusion is a procedure that involves joining two or more adjacent vertebrae to restrict movement of the vertebrae with respect to one another. For a number of reasons, spinal fixation devices are used in spine surgery to align and/or secure a desired relationship between adjacent vertebral bodies. Such devices typically include a spinal fixation element, such as a relatively rigid fixation rod that is coupled to adjacent vertebrae by attaching the fixation element to various bone fixation elements, such as hooks, bolts, wires, screws, etc. The fixation elements can have a predetermined contour, and once installed, the fixation element holds the vertebrae in a desired spatial relationship preferably until desired healing or spinal fusion takes place.

Dynamic fixation elements are desirable, at least in part, because they absorb shock, for example, in the extension and compression of the spine. In addition, the removal of bone structure, such as facet joints or laminae, result in instabilities of the motion segments of the spine. Consequently, a fixation system should stabilize the motion segment in antero-posterior translation as well as in axial rotation. Both motion patterns result in shear stress within the spinal fixation element of the fixation system. This is especially important in elderly patients, where bone quality is sometimes compromised, becoming sclerotic or osteoporotic.

It is desirable to have a dynamic fixation system that provides constraints regarding shear stresses and improves stabilization without limiting the system's range of motion in flexion. It is also desirable to provide a system comprising a low number of components to reduce the complexity of the assembly.

The present invention is related to a dynamic or flexible stabilization system that can be used for stabilization of a portion of a patient's spine. The dynamic stabilization system can be implanted to the patient's spine using pedicle screws as is currently performed in conventional pedicle screw systems.)

Known dynamic stabilization systems (WO 01/56489A; US 2008/0097434A; WO 2006/066053A; EP0677277A; US 2007/0016200A; NO 2007/103404A) include a first fixation element, an a second fixation element such as for example bone screws mounted to a first and second vertebrae, respectively, an elongated fixation element such as a rod, including a first portion and a second portion, the first portion mounted to the first fixation element and the second portion mounted to the second fixation element, and a dampening element mounted between the first and second portions, the dampening element including a plurality of segments and a plurality of bridging elements connecting the plurality of segments to permit movement of the first portion relative to the second portion.

According to the invention, the segments and the bridging elements connecting the segments are arranged in a plane perpendicular to the longitudinal axis of the elongated fixation element. In one exemplary embodiment, the system preferably comprises multiple dampening elements between the first and second fixation elements. The segments and bridging element may be formed using Electrical Discharge Machining to create concentric or almost concentric segments separated by said bridging element. In another embodiment, the segments additionally comprise diagonal grooves that are separated by slots which are created through the process of machining the center of the dampening element.

In one exemplary embodiment, the bone fixation elements are bone screws and comprise a channel in their head for receiving the elongated fixation element in an implanted position. In another preferred embodiment, multiple dampening elements are located in series along the elongated fixation element between a first fixation element and a second fixation element.

### BRIEF DESCRIPTION OF THE SEVERAL VIEWS OF THE DRAWINGS

The foregoing summary, as well as the following detailed description of preferred embodiments of the application, will be better understood when read in conjunction with the appended drawings. For the purposes of illustrating the dynamic stabilization system of the present application, there is shown in the drawings preferred embodiments. It should be understood, however, that the application is not limited to the precise arrangement, structures, features, embodiments, aspects, and instrumentalities shown, and the arrangements, structures, features, embodiments, aspects and instrumentalities shown may be used singularly or in combination with other arrangements, structures, features, embodiments, aspects and instrumentalities. In the drawings:

Fig. 1 is a top perspective view of a dynamic stabilization system according to a first preferred embodiment of the present invention mounted to a spine;

Fig. 2 is a side perspective view of a dynamic stabilization system of Fig. 1;

Fig. 3 is a side perspective view of a dampening element of the dynamic stabilization system of Fig. 1;

Fig. 4 is an alternate side perspective of the dampening element of the dynamic stabilization system of Fig. 1;

Fig. 5 is a cross-sectional view of the damping element of Fig. 3, taken along line 5-5 of Fig. 3;

Fig. 6 is side elevational view of multiple dampening elements on an elongated fixation element according to a second preferred embodiment of the present invention;

Fig. 7 is a cross-sectional view of the elongated fixation element of Fig. 6, taken along line 7-7 of Fig. 6;

Fig. 8 is side perspective view of a dampening element on an elongated fixation element according to a third preferred embodiment of the present invention; and

Fig. 9 is a cross-sectional view of the elongated fixation element of Fig. 8, taken along line 9-9 of Fig. 8.

### DETAILED DESCRIPTION OF THE INVENTION

Certain terminology is used in the following description for convenience only and is not limiting. The words "right", "left", "top" and "bottom" designate directions in the drawings to which reference is made. The words "inwardly" and "outwardly" refer to directions toward and away from, respectively, the geometric center of the dynamic stabilization system, the damping element of the elongated fixation element and designated parts thereof. The words, "anterior", "posterior", "superior", "interior", "lateral", "sagittal", "axial", "coronal" and related words and/or phrases designate preferred positions and orientations in the human body to which reference is made and are not meant to be limiting. The terminology includes the above-listed words, derivatives thereof and words of similar import.

Certain exemplary embodiments will now be described with reference to the drawings. In general, such embodiments relate to a dynamic stabilization system, by way of non-limiting example, a dynamic stabilization system for posterior spinal fixation. As will be described in greater detail below, the dynamic stabilization system may include one or more dynamic bone fixation elements for flexibly connecting an elongated fixation element to two or more bones. The dynamic stabilization system preferably further includes a flexible dampening element which preferably permits the elongated fixation element to move with respect to the bone fixation element and hence with respect to the bone affixed thereto.

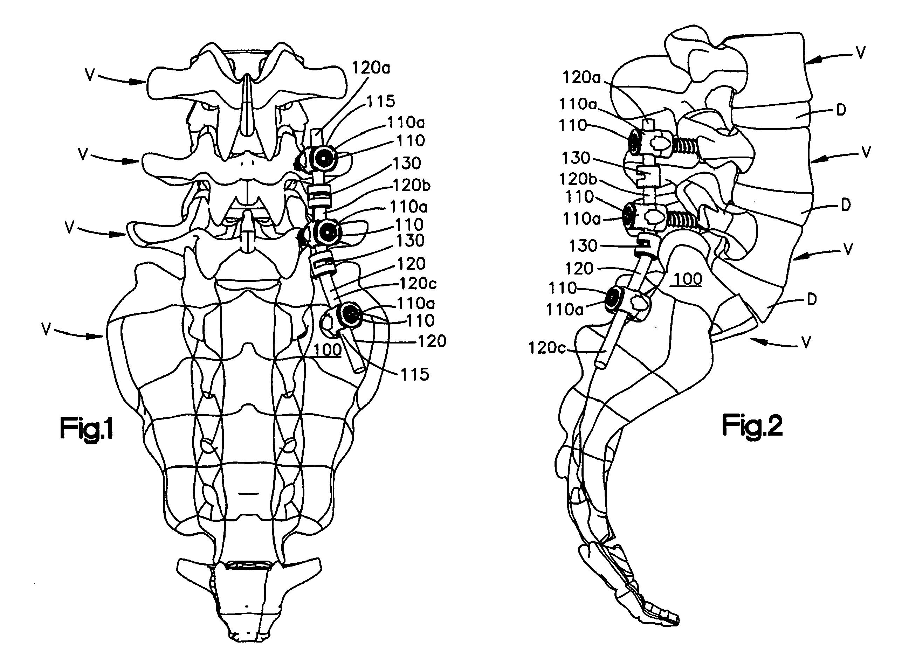

Referring to Figs. 1 and 2, a first preferred embodiment of a dynamic stabilization system 100 of the present invention includes a plurality of bone fixation elements 110, an elongated fixation element (shown here as an elongated rod) 120 and a dampening element 130. The bone fixation elements or bone screws 110 are configured for securing the elongated fixation element 120 to a patient's bone, preferably a patient's vertebra V. The dampening element 130 permits movement of the bone fixation elements or bone screws 110 and the associated vertebrae V with respect to each other.

The bone fixation elements 110 may be in the form of poly-axial or mono-axial pedicle or bone screws, hooks (both mono-axial and poly-axial) including pedicle hooks, transverse process hooks, sublaminar hooks, or other fasteners, clamps or implants or any other fastening device now or hereafter known in the art. The elongated fixation element 120 may be in the form of a longitudinal rod, bone plate, or any other device now or hereafter known in the art that is generally rigid to secure at least two bone fixation elements 110 together. It will be recognized by one having ordinary skill in the art that the elongated fixation element 120 may include, but is limited to, a solid rod, a non-solid rod, a polymeric flexible or dynamic rod, etc. The dynamic stabilization system 100 of the present application is preferably not limited to use with any particular type of bone fixation element 110 or elongated fixation element 120. The bone fixation element 120 preferably includes a first portion 120a and a second portion 120b that are mounted to the bone fixation elements 110 in an implanted position. The bone fixation element 120 of the first preferred embodiment also includes a third portion 120c that is mounted to a third bone fixation element 110 in the implanted position. The bone fixation element 120 is not limited to inclusion of the first, second and third portions 120a, 120b, 120c and may include additional portions for mounting to additional bone fixation elements 110, but preferably include at least the first and second portions 120a, 120b for mounting to two bone fixation elements 110.

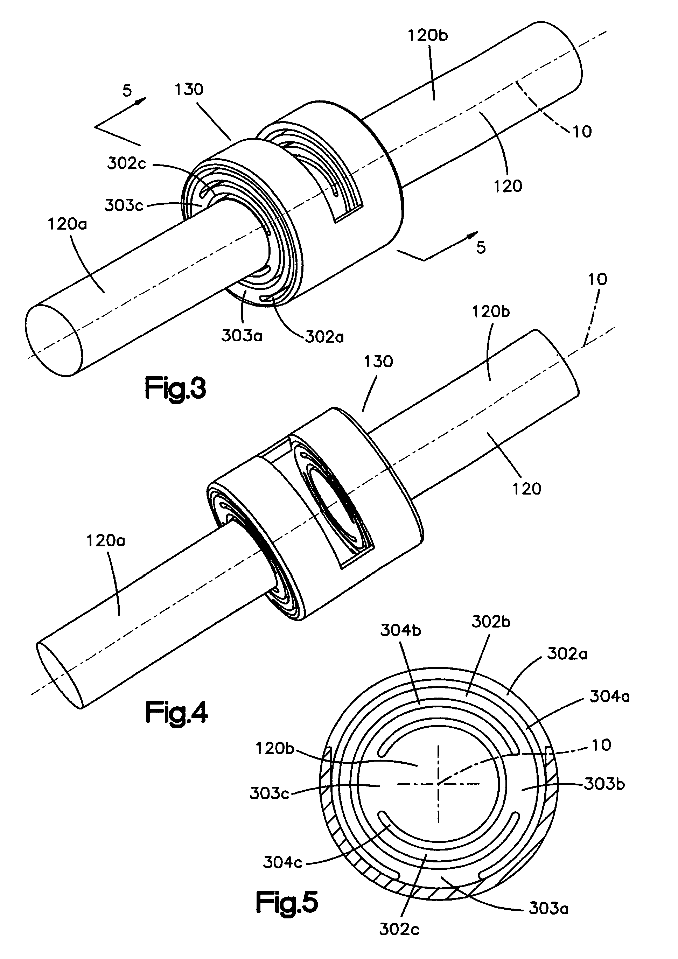

Referring to Figs. 3-5, the preferred dampening element 130 is constructed by cutting or forming a plurality of concentric or almost concentric narrow grooves 304a, 304b, 304c, bridged together in a horizontal plane by bridging elements 303a, 303b, 303c and separated by concentric segments 302a, 302b, 302c for damping motion between the first and second portions 120a, 120b of the elongated fixation element 120. These concentric or almost concentric grooves 304a, 304b, 304c are preferably constructed using Electrical Discharge Machining ("EDM"), although other machining methods can be utilized. In EDM, a series of rapidly recurring electrical discharges or sparks are passed between two electrodes separated by a dielectric liquid. The electrical discharges are passed between one of the electrodes and the dampening element 130. Through this process small amounts of metal or, potentially, a polymer, a ceramic or composite material, are removed from the dampening element 130. The repetitive discharges create a set of successively deeper indentations forming the concentric or almost concentric grooves 304a, 304b, 304c in the dampening element 130, as well as defining the bridging elements 303a, 303b, 303c and the concentric segments 302a, 302b, 302c. The EDM processing method permits integral construction of the elongated fixation element 120 and the dampening element 130 from a single piece of material, but the first preferred embodiment of the dynamic stabilization system 100 is not so limited. For example, the first and second portions 120a, 120b and the dampening element 130 may be constructed of separate pieces of material and subsequently joined together in a manufacturing assembly process via welding, a threaded connection, adhesive bonding or alternative joining methods to attach the first and second portions 120a, 120b to the dampening element 130. For example, the first portion 120a may be threadably mounted to a first side of the dampening element 130 along a longitudinal axis 10 and the second portion 120b may be threadably mounted to a second side of the dampening element 130 along the longitudinal axis 10.

The dampening element 130 and the elongated fixation element 120 may be constructed of at least one of the group of metals consisting of Ti-Mo, CoCr, a fatigue resistant biocompatible metal, Titanium, Titanium alloy, Cobalt-Chromium alloy, a biocompatible polymer, a biocompatible mixture of polymers, Nitinol, shape memory material, ceramic, and a composite material. The concentric grooves 304a, 304b, 304c and the concentric segments 302a, 302b, 302c are disclosed in the first preferred embodiment as being generally concentric about the longitudinal axis 10 of the elongated fixation element 120, but are not so limited. The grooves 304a, 304b, 304c and segments 302a, 302b, 302c may be otherwise formed in the damping element 130 in a non-concentric pattern, for example, to adapt the damping resistance of the damping element 130 in specific directions or about specific axes to yield damping properties that are desirable to the designer or user.

The segments 302a-c are preferably continuously closed shapes, i.e., the segments 302a-c form a closed space and, preferably, are shaped as closed rings or O-shaped rings. The segments 302a-c, however, may be of any shape, including, but not limited to, circular, rectangular, oval, C-shaped, horseshoe, triangular, octagonal, U-shaped or kidney shaped. The number of segments 302a-c and bridging elements 303a-c may vary and are not limited to any particular number, value or range. Although the first preferred embodiment includes the substantially closed concentric segments 302a-c, the segments 302a-c may also be open, forming, for example, a C-shaped segment, horseshoe shaped, or any other suitable shape.

The segments 302a-c of dampening element 130 preferably allow for movement in at least six degrees of freedom between the first and second portions 120a, 120b of the elongated fixation element 120, including flexion, extension, lateral bending, axial rotation, horizontal shifting, and dampening of the spine. The deflection and translation of the segments 302a-c and bridging elements 303a-c in response to compressive forces creates a dampening effect in the dynamic stabilization system 100. The plurality of segments 302a-c and bridges 303a-c define the concentric or almost concentric narrow grooves 304a-c that are oriented in such a way to control and/or limit anterior and posterior shifts, medial and lateral shifts, axial rotation (both clockwise and counter clockwise), anterior and posterior flexion and extension, lateral movement and combinations thereof. A dampening effect will therefore be possible due to the freedom in the vertical plane.

The bridging elements 303a-c of the first preferred embodiment connect the segments 302a-c and may permit and provide resistance to relative movement of the segments 302a-c and the dynamic stabilization system 100. The resistance to relative movement of the segments 302a-c, and consequently the dynamic stabilization system 100, may be varied and controlled by altering the number, height, material, thickness (or width), shape, or other properties of the segments 302a-c and, consequently, the size, shape and/or thickness of the bridging elements 303a-c and the grooves 304a-c. In addition, the resistance to relative movement of the segments 302a-c and, consequently, the dynamic stabilization system 100, may also be varied and controlled by altering the number, width, thickness, material, shape, or other properties of the bridging elements 303a-c. Resistance to the relative movement of the segments 302a-c, and consequently, the dynamic stabilization system 100, may further be varied and controlled by increasing the number of bridging elements 303a-c that connect two adjacent segments 302a-c, such as the first segments 302a and the second segment 302b. For example, multiple bridging elements 303a-c may be used to connect adjacent segments 302a-c to vary the resistance to relative movements between the segments 302a-c. Although the first preferred embodiment of the dynamic stabilization system 100 includes three bridging elements 302a-c aligned along a medial/lateral axis, the bridging elements 303a-c may be positioned in any manner between the segments 302a-c of the dampening element 130. In the first preferred embodiment, the bridging elements 303a-c generally prevent contact between the separate segments 302a-c under biomechanically relevant load. Accordingly, the lack of contact between the separate segments 302a-c generally limits metal-debris that could result if the segments 302a-c rubbed against each other during use.

In an implanted position, the dynamic stabilization system 100 may engage one or more vertebrae V via the bone fixation elements 110, which engage one or more elongated fixation elements 120 at its first and/or second portions 120a, 120b so that the dynamic stabilization system 100 dynamically stabilizes the vertebrae V with respect to one another. The dynamic stabilization system 100 may be used in a spinal construct in combination with an intervertebral implant (not shown) for fusing adjacent vertebrae V or dynamically replacing an intervertebral disc D between adjacent vertebrae V. The dynamic stabilization system 100 of the first preferred embodiment may permit the vertebrae V to settle (e.g. compress) over time, thus facilitating fusion between the intervertebral implant and the adjacent vertebrae V. Alternatively, the dynamic stabilization system 100 may be used in connection with an articulating intervertebral implant (not shown) or any other implant known in the art, or none at all. Moreover, the amount and type of movement that is permitted by the dynamic stabilization system 100 can be tailored for individual patients. For example, for patients with less severe pathologies (e.g., better bone structure), a less stiff system may be desirable to permit additional movement. Likewise, for patients with severely degenerated discs, a stiffer system may be desirable to permit less or no movement. Moreover, the elongated fixation element 120 can be provided in different degrees of softness to enhance stress-shielding, especially for patients with osteoporotic bones. The elongated fixation element 120 can further be adapted to a rigid type device by blocking or binding the dampening element 130. In addition, the dynamic stabilization system 100 may be configured such that the first portion 120a is engaged by a pair of bone fixation elements 110 secured to adjacent vertebrae V (not shown) to generally hold the adjacent vertebrae V in place and promote fusion, while the second portion 120b is engaged to a third bone fixation element 110 secured to a third vertebra V to permit movement between this third vertebra V and the pair of vertebrae V secured to the first portion 120a. Accordingly, the dynamic stabilization system 100 may be configured to promote fusion of selected pairs of vertebrae V, generally in combination with a fusion implant to replace a disc D, and to preserve motion in an adjacent disc D, at the spinal motion segment spanned by the damping element 130, with or without the combination of a total disc replacement implant.

Referring to Figs. 1 and 2, the individual vertebrae V are preferably stabilized posteriorly using the dynamic stabilization system 100 of the first preferred embodiment. Specifically, the bone fixation elements 110 are secured into three vertebrae V from the posterior direction and are preferably mounted in pedicles P of the vertebrae V. Heads of the bone fixation elements 110 each preferably have a U-shaped channel or a rod-receiving channel 115, for accommodating and/or receiving the first, second and third portions 120a, 120b, 120c of the elongated fixation element 120, respectively. The dynamic stabilization system 100 is preferably capable of being fixed to the elongated fixation element 120 by securing the first, second and third portions 120a-c in the channels 115 by, for example, a closure cap, set screw or locking cap 110a, as generally understood by one of ordinary skill in the art. In this manner, the spine of the patient can be stabilized.

In the implanted position, as the attached vertebrae V move, the movement and associated loads are transferred from the vertebrae V to the dynamic stabilization system 100. In this manner, the dynamic stabilization system 100 permits the attached vertebrae V to move with respect to one another such that the patient does not lose all motion at the impacted motion segment or segments of the spine. The combination of the bone fixation elements 110, elongated fixation element 120 and dampening elements 130 may absorb some or all of the movement (e.g., translation, articulation, rotational (e.g., twisting), etc.) and associated loads and/or stresses and portions of the loads and/or stresses are also carried by the patient's spinal anatomy.

In the implanted position, the length of the dynamic stabilization system 100 will depend on the size and number of vertebrae V being secured or supported. For example, the length of the elongated fixation element 120 may be up to one meter (1 m) long, if the patient's entire spine is being secured and/or instrumented. As will be generally understood by one of ordinary skill in the art, the diameter of the elongated fixation element 120 and dampening elements 130 will be sized to absorb the expected loads. Thus, the dynamic stabilization system 100 of the first preferred embodiment is shown as being mounted in and is preferably sized for use in the lumbar region of the spine and will typically have a larger diameter than one sized for use in the thoracic or cervical regions.

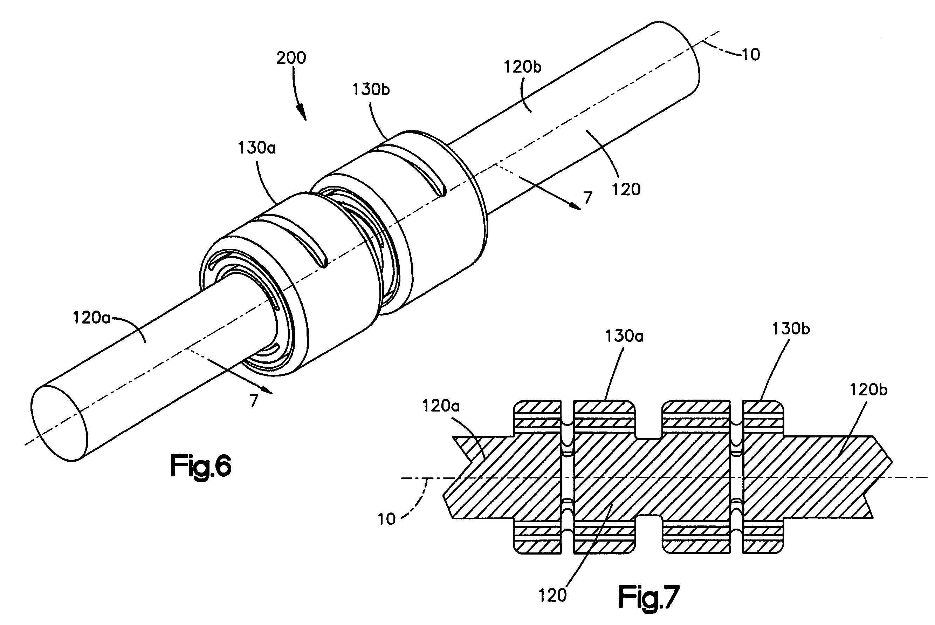

Referring to Figs. 6 and 7, in a second preferred embodiment, multiple dampening elements 130a, 130b are arranged or mounted in series between the first and second portions 120a, 120b of the elongated fixation element 120. This may be particularly beneficial for multiple-level constructs. Using multiple dampening elements 130a, 130b in succession between fixation elements 110 and the first and second portions 120a, 120b preferably allows more flexibility for the elongated fixation element 120 and movement for the dynamic stabilization system 200 of the second preferred embodiment. The dampening elements 130a, 130b can be spaced closer or farther apart to increase or decrease the dampening effect, as desired by the designer or user.

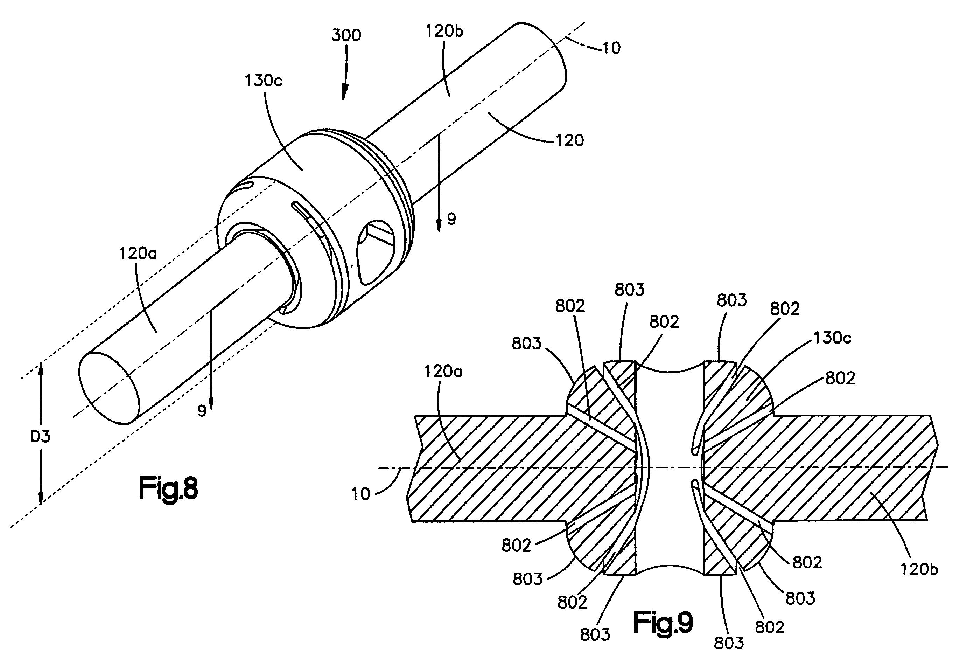

Referring to Figs. 8 and 9, in a third preferred embodiment, in addition to the concentric segments 302a-c separated by the grooves 304a-c and joined by the bridges 303a-c, a dampening element 130c of the third preferred embodiment may be constructed by machining diagonally through the center of the dampening element 130c. Machining the dampening element 130c in this manner forms grooves 802 that are separated by slots 803 in multiple locations throughout the dampening element 130c. In the third preferred embodiment, the creation of the grooves 802 and the slots 803 preferably results in a smaller diameter D₃ for the dampening element 130c and increased flexibility of the dynamic stabilization system 300. The dampening element 130 of the third preferred embodiment preferably attains increased movement in at least six degrees of motion, including flexion, extension, lateral bending, axial rotation, horizontal shifting, and dampening via construction of the dampening element 130c with the grooves 802 and slots 803. As discussed above with regard to the dampening elements 130a, 130b of the first and second preferred embodiments, the dampening element 130c of the third preferred embodiment can be situated in series along the elongated fixation element 120 between the first and second portions 120a, 120b to create an increased dampening effect. The grooves 802 and slots 803 of the third preferred embodiment may be constructed utilizing the above-described EDM process and the dampening element 130c may be integrally constructed from a single piece of material with the first and second portions 120a, 120b. Alternatively, the grooves 802 and slots 803 may be constructed utilizing high speed machining techniques and the dampening element 130c may be separately constructed from the first and second portions 120a, 120b and the first and second portions 120a, 120b may be subsequently joined to the dampening element 130c.

As will be appreciated by those skilled in the art, any or all of the components described herein such as, for example, the bone fixation elements 110, the elongated fixation elements 120 and the dampening elements 130, 130a-c may be provided in sets or kits so that the surgeon may select various combinations of components to perform a fixation procedure and create a stabilization system which is configured specifically for the particular needs/anatomy of a patient. It should be noted that one or more of each component may be provided in a kit or set. In some kits or sets, the same device may be provided in different shapes and/or sizes (e.g., multiple bone fixation elements 110, elongated fixation elements 120 and/or dampening elements 130, 130a-c of different sizes). In the first preferred embodiment, each segment 302a-c is approximately between two tenths and two millimeters (0.2 mm - 2.0 mm) in width and between eight and thirty millimeters (8 mm - 30 mm) in depth. Each concentric segment 302a-c preferably allows at least thirty degrees (30°) of movement of the dynamic stabilization system 100 of the first preferred embodiment relative to the spine.

Referring to Figs. 1-9, in use, an incision is formed in a patient's back to gain access to the spine and, particularly, the vertebrae V at the motion segments that will be instrumented utilizing the preferred dynamic stabilization system 100, 200, 300. An appropriate number of the bone fixation elements 110 are mounted to the appropriate vertebrae V, preferably in the pedicles P of the vertebrae V. The elongated fixation element 120 is arranged relative to the mounted bone fixation elements 110 such that the first, second and/or third portions 120a, 120b, 120c are positioned within the channels 115 of the bone fixation elements 110 and the dampening elements 130, 130a, 130b, 130c are positioned between the bone fixation elements 110 at spinal levels where dynamic fixation between vertebrae V is desired. The elongated fixation element 120 is then fixed to the bone fixation elements 110, by securing locking caps 110a to the bone fixation elements 110 to fix the first, second and or third segments 120a-c in the channels 115. Instrumentation is removed from the incision and the incision is closed. As was described above, the first segment 120a may be mounted between two bone fixation elements 110a and 110b to generally fix the position of the bone fixation elements 110a and 110b relative to each other, while a third bone fixation element 110c may be fixed to the second segment 120b to permit dampened movement between the two fixed bone fixation elements 110a and 110b and the third bone fixation element 110c.

Those skilled in the art will recognize that system of the present invention has many applications, may be implemented in many manners and, as such is not to be limited by the foregoing embodiments and examples. Any number of the features of the different embodiments described herein may be combined into one single embodiment and alternate embodiments having fewer than or more than all of the features herein described are possible. Functionality may also be, in whole or in part, distributed among multiple components, in manners now known or to become known. Moreover, the scope of the present invention covers conventionally known and features of those variations and modifications through the components described herein as would be understood by those skilled in the art. It is the intention, therefore, to be limited only as indicated by the scope of the claims appended hereto.

It is also to be understood that the following claims are intended to cover all of the generic and specific features of the invention herein described and all statements of the scope of the invention, which, as a matter of language, might be said to fall therebetween.

It will be appreciated by those skilled in the art that changes could be made to the embodiments described above without departing from the broad inventive concept thereof. It is understood, therefore, that this invention is not limited to the particular embodiments disclosed, but it is intended to cover modifications within the scope of the present invention as defined by the appended claims.

## Claims

1. A dynamic stabilization system for mounting to a first vertebra (V) and a second vertebra (V) of a spine, the dynamic stabilization system comprising:
a first fixation element (110) and a second fixation element (110), said fixation elements comprising bone screws, mounted to the first and second vertebrae (V), respectively;
an elongated fixation element (120) including a first portion (120a) and a second portion (120b), the first portion mounted to the first fixation element and the second portion mounted to the second fixation element;
a dampening element (130) located between the first and second portions (120a, 120b), the dampening element including a plurality of segments (302a-302c) and a plurality of bridging elements (303a-303c) connecting the plurality of segments to permit movement of the first portion (102a) relative to the second portion (102b);
**characterized in that** the segments (302a-302c) and the bridging elements (303a-303c) connecting the segments are arranged in a plane perpendicular to the longitudinal axis (10) of the elongated fixation element (120).

2. The system of claim 1, wherein the plurality of segments (302a-302c) is comprised of semi-circular shaped rings or closed rings.

3. The system of claim 1, wherein the segments (302a-302c) are open, in particular C-shaped or horseshoe-shaped.

4. The system of any of claims 1 to 3, wherein the segments (302a-302c) are concentric about the longitudinal axis (10), or almost concentric.

5. The system of any of claims 1 to 4, wherein the elongated fixation element (120) and/or the dampening element (130) is constructed of at least one of the group consisting of Ti-Mo, CoCr, a fatigue resistant biocompatible metal, Titanium, Titanium alloy, Cobalt-Chromium alloy, a biocompatible polymer, a biocompatible mixture of polymers, Nitinol, shape memory material, ceramic, and a composite material.

6. The system of any of claims 1 to 5, wherein the system includes multiple dampening elements (130a, 130b) between the first and second fixation elements (110).

7. The system of claim 6, wherein the multiple dampening elements (130a, 130b) are arranged in series.

8. The system of any of claims 1 to 7, wherein the elongated fixation element (120) is comprised of a rod.

9. The system of any of claims 1 to 8, wherein the first and second portions (120a, 120b) and the dampening element (130) are integrally constructed from a single piece of material.

10. The system of any of claims 1 to 8, wherein the first and second portions (120a, 120b) and the dampening element (130) are constructed from at least two pieces of material, the first portion (120a) being threadably mounted to a first side of the dampening element (130) along the longitudinal axis (10) and the second portion (120b) being threadably mounted to a second side of the dampening element (130) along the longitudinal axis (10).

11. The system of any of claims 1 to 5, wherein the first and second fixation elements (110) each include a channel for insertion of the elongated fixation element in an implanted position, preferably a U-shaped channel.

12. The system of claim 11, wherein
the first fixation element (120a) includes a first head portion having the first insertion channel;
the second fixation element (120b) includes a second head portion having the second insertion channel;
the first portion (120a) of the fixation element (120) to be mounted in the first insertion channel and the second portion (120b) of the fixation element (120) to be mounted in the second insertion channel in an implanted position.

13. The system of any of claims 1 to 12, wherein the plurality of segments (302a-302c) is constructed by the process of electrical discharge machining.

14. The system of any of claims 1 to 12, wherein the plurality of segments (302a-302c) is comprised of diagonal grooves.

15. The system of any of claims 1 to 14, wherein each of the bridging elements connects two adjacent segments, respectively, wherein preferably adjacent segments (302a-302c) are connected by multiple bridges.

## Patentansprüche

1. Dynamisches Stabilisierungssystem zum Anbringen an einem ersten Wirbel (V) und einem zweiten Wirbel (V) einer Wirbelsäule, wobei das dynamische Stabilisierungssystem umfasst:
ein erstes Fixierelement (110) und ein zweites Fixierelement (110), angebracht an dem ersten bzw. zweiten Wirbel (V), wobei die Fixierelemente Knochenschrauben umfassen,
ein längliches Fixierelement (120), welches einen ersten Teil (120a) und einen zweiten Teil (120b) enthält, wobei der erste Teil an dem ersten Fixierelement angebracht ist und der zweite Teil an dem zweiten Fixierelement angebracht ist,
ein Dämpfungselement (130), welches sich zwischen dem ersten und zweiten Teil (120a, 120b) befindet, wobei das Dämpfungselement eine Mehrzahl von Segmenten (302a-302c) und eine Mehrzahl von Brückenelementen (303a-303c) enthält, welche die Mehrzahl von Segmenten verbinden, um eine Bewegung des ersten Teils (102 a) relativ zu dem zweiten Teil (102 b) zu erlauben,
**dadurch gekennzeichnet, dass** die Segmente (302a-302c) und die die Segmente verbindenden Brückenelemente (303a-303c) in einer Ebene senkrecht zu der Längsachse (10) des länglichen Fixierelements (120) angeordnet sind.

2. System nach Anspruch 1, wobei die Mehrzahl der Segmente (302a-302c) halbkreisförmige Ringe oder geschlossene Ringe umfasst.

3. System nach Anspruch 1, wobei die Segmente (302a-302c) offen sind, insbesondere C-förmig oder hufeisenförmig.

4. System nach einem der Ansprüche 1 bis 3, wobei die Segmente (302a-302c) konzentrisch oder fast konzentrisch um die Längsachse (10) sind.

5. System nach einem der Ansprüche 1 bis 4, wobei das längliche Fixierelement (120) und/oder das Dämpfungselement (130) aus zumindest einem der Gruppe, bestehend aus Ti-Mo, CoCr, einem ermüdungsresistenten biokompatiblen Material, Titan, einer Titanlegierung, einer Kobalt-Chrom-Legierung, einem biokompatiblen Polymer, einer biokompatiblen Mischung von Polymeren, Nitinol, einem Formgedächtnismaterial, Keramik und einem Verbundwerkstoff, aufgebaut sind.

6. System nach einem der Ansprüche 1 bis 5, wobei das System mehrere Dämpfungselemente (130a, 130b) zwischen dem ersten und zweiten Fixierelement (110) enthält.

7. System nach Anspruch 6, wobei die mehreren Dämpfungselemente (130a, 130b) in Reihe angeordnet sind.

8. System nach einem der Ansprüche 1 bis 7, wobei das längliche Fixierelement (120) eine Stange umfasst.

9. System nach einem der Ansprüche 1 bis 8, wobei der erste und zweite Teil (120a, 120b) und das Dämpfungselement (130) einstückig aus einem einzigen Materialstück aufgebaut sind.

10. System nach einem der Ansprüche 1 bis 8, wobei der erste und zweite Teil (120a, 120b) und das Dämpfungselement (130) aus zumindest zwei Materialstücken aufgebaut sind, wobei der erste Teil (120a) mittels Gewinde an einer ersten Seite des Dämpfungselements (130) entlang der Längsachse (10) angebracht ist und der zweite Teil (120b) mittels Gewinde an einer zweiten Seite des Dämpfungselements (130) entlang der Längsachse (10) angebracht ist.

11. System nach einem der Ansprüche 1 bis 5, wobei das erste und zweite Fixierelement (110) jeweils einen Kanal zum Einführen des länglichen Fixierelements in einer implantierten Position enthalten, vorzugsweise einen U-förmigen Kanal.

12. System nach Anspruch 11, wobei
das erste Fixierelement (120a) einen ersten Kopfteil enthält, welcher den ersten Einführkanal aufweist,
das zweite Fixierelement (120b) einen zweiten Kopfteil enthält, welcher den zweiten Einführkanal aufweist,
in einer implantierten Position der erste Teil (120a) des Fixierelements (120) in dem ersten Einführkanal anzubringen ist und der zweite Teil (120b) des Fixierelements (120) in dem zweiten Einführkanal anzubringen ist.

13. System nach einem der Ansprüche 1 bis 12, wobei die Mehrzahl der Segmente (302a-302c) mittels des Funkenerosionsbearbeitungsprozesses aufgebaut ist.

14. System nach einem der Ansprüche 1 bis 12, wobei die Mehrzahl der Segmente (302a-302c) diagonale Nuten umfasst.

15. System nach einem der Ansprüche 1 bis 14, wobei jedes der Brückenelemente entsprechende zwei benachbarte Segmente verbindet, wobei vorzugsweise benachbarte Segmente (302a-302c) durch mehrere Brücken verbunden sind.

## Revendications

1. Système de stabilisation dynamique destiné à un montage sur une première vertèbre (V) et une deuxième vertèbre (V) d'une colonne vertébrale, le système de stabilisation dynamique comprenant :
un premier élément de fixation (110) et un second élément de fixation (110), lesdits éléments de fixation comprenant respectivement des vis à os, montées sur les première et deuxième vertèbres (V) ;
un élément de fixation oblong (120) incluant une première partie (120a) et une seconde partie (120b), la première partie étant montée sur le premier élément de fixation et la seconde partie étant montée sur le second élément de fixation, et
un élément amortisseur (130) placé entre les première et secondes parties (120a, 120b), l'élément amortisseur incluant une pluralité de segments (302a-302c) et une pluralité d'éléments de pontage (303a-303c) reliant la pluralité de segments afin de permettre le mouvement de la première partie (102a) par rapport à la seconde partie (102b) ;
**caractérisé en ce que** les segments (302a-302c) et les éléments de pontage (303a-303c) reliant les segments sont agencés suivant un plan perpendiculaire à l'axe longitudinal (10) de l'élément de fixation oblong (120).

2. Système selon la revendication 1, dans lequel la pluralité de segments (302a-302c) est constituée d'anneaux présentant une forme semi-circulaire ou d'anneaux fermés.

3. Système selon la revendication 1, dans lequel les segments (302a-302c) sont ouverts, présentant en particulier une forme en C ou une forme en fer à cheval.

4. Système selon l'une quelconque des revendications 1 à 3, dans lequel les segments (302a-302c) sont concentriques autour de l'axe longitudinal (10) ou quasiment concentriques.

5. Système selon l'une quelconque des revendications 1 à 4, dans lequel l'élément de fixation oblong (120) et/ou l'élément amortisseur (130) est construit à partir d'au moins un élément du groupe consistant en du Ti-Mo, du CoCr, un métal biocompatible résistant à la fatigue, du titane, un alliage de titane, un alliage cobalt-chrome, un polymère biocompatible, un mélange biocompatible de polymères, du Nitinol, un matériau à mémoire de forme, une céramique ou un matériau composite.

6. Système selon l'une quelconque des revendications 1 à 5, dans lequel le système inclut de multiples éléments amortisseurs (130a, 130b) entre les premier et second éléments de fixation (110).

7. Système selon la revendication 6, dans lequel les multiples éléments amortisseurs (130a, 130b) sont agencés en série.

8. Système selon l'une quelconque des revendications 1 à 7, dans lequel l'élément de fixation oblong (120) est constitué d'une tige.

9. Système selon l'une quelconque des revendications 1 à 8, dans lequel les première et seconde parties (120a, 120b) et l'élément amortisseur (130) sont construits d'un seul tenant à partir d'une pièce métallique unique.

10. Système selon l'une quelconque des revendications 1 à 8, dans lequel les première et seconde parties (120a, 120b) et l'élément amortisseur (130) sont construits à partir d'au moins deux pièces métalliques, la première partie (120a) étant montée par filetage sur un premier côté de l'élément amortisseur (130) le long de l'axe longitudinal (10), et la seconde partie (120b) étant montée par filetage sur un second côté de l'élément amortisseur (130) le long de l'axe longitudinal (10).

11. Système selon l'une quelconque des revendications 1 à 5, dans lequel les premier et second éléments de fixation (110) incluent chacun un canal pour l'insertion de l'élément de fixation oblong dans une position implantée, de préférence un canal en forme de U.

12. Système selon la revendication 11, dans lequel
le premier élément de fixation (120a) inclut une première partie de tête présentant le premier canal d'insertion ;
le second élément de fixation (120b) inclut une seconde partie de tête présentant le second canal d'insertion ;
la première partie (120a) de l'élément de fixation (120) devant être montée dans le premier canal d'insertion et la seconde partie (120b) de l'élément de fixation (120) devant être montée dans le second canal d'insertion dans une position implantée.

13. Système selon l'une quelconque des revendications 1 à 12, dans lequel la pluralité de segments (302a-302c) est construite par le procédé d'usinage par électroérosion.

14. Système selon l'une quelconque des revendications 1 à 12, dans lequel la pluralité de segments (302a-302c) comprend des rainures diagonales.

15. Système selon l'une quelconque des revendications 1 à 14, dans lequel chacun des éléments de pontage relie respectivement deux segments adjacents, dans lequel des segments adjacents (302a-302c) sont de préférence reliés par de multiples ponts.
